# EUROPEAN PATENT APPLICATION

(11) **EP 4 803 005 A1**
(43) Date of publication of application: **09.09.2026**
(21) Application number: 24886359.9
(22) Date of filing: 01.11.2024
(51) Int. Cl.: A61B 6/00, A61B 6/40

(54) **X-RAY IMAGING APPARATUS**

(30) Priority: 01.11.2023 KR 20230149168
(71) Applicant: Vatech Co., Ltd., Hwaseong-si, Gyeonggi-do 18449 (KR); Vatech Ewoo Holdings Co., Ltd., Hwaseong-si, Gyeonggi-do, 18449 (KR)
(72) Inventor: CHOI, Sung Il, Hwaseong-si Gyeonggi-do 18449 (KR); JO, Tae Young, Hwaseong-si Gyeonggi-do 18449 (KR)
(74) Representative: Lorenz & Kollegen
(86) International application number: PCT/KR2024/017062
(87) International publication number: WO 2025/095688

(57) **Abstract**

Provided is an X-ray imaging apparatus which utilizes characteristic values of a field emission type X-ray generator and an X-ray detector so as to perform X-ray imaging through the high-speed pulse driving of X-rays, thereby providing an image of the same quality at a relatively low dose, or providing an image of relatively high quality at an equal or lower dose. The X-ray imaging apparatus according to the present invention comprises: an imaging unit including an X-ray generator, which irradiates a subject with X-rays, and an X-ray detector, which receives the X-rays that passed through the subject, so as to generate frame-by-frame projection data; an input unit into which imaging information including at least one from among tube voltage, tube current and an irradiation time is input; a storage unit for storing generator irradiation information including a rising time and/or a falling time of the X-ray generator, detector detection information including a frame rate and/or a readout time of the detector, and dose-pulse-image quality relationship information about the relationship between the X-ray dose, number of X-ray pulses and image quality of an X-ray image; and a control unit for calculating, according to the imaging information, the number of X-ray pulses for X-ray imaging of the subject by using the generator irradiation information, the detector detection information and the dose-pulse-image quality relationship information.

## Description

### Technical Field

The present disclosure relates to an X-ray imaging apparatus. More particularly, the present disclosure relates to an X-ray imaging apparatus configured to drive an X-ray generator in an on/off pulse manner.

### Background Art

In industrial fields including the medical field, an X-ray imaging apparatus refers to an apparatus which detects, by using an X-ray detector, X-rays emitted from an X-ray generator and transmitted through a subject, and which constructs an X-ray image on the basis of detected electrical signals. X-rays are attenuated and transmitted at different attenuation rates according to a material along a propagation path thereof, and are converted into electrical signals by a photoelectric effect when the X-rays reach the X-ray detector. The X-ray imaging apparatus provides information on an interior of a subject as an X-ray image by using electrical signals reflecting accumulated attenuation along the propagation path of X-rays.

Recently, research and development of an electric field emission type X-ray source using nanostructures such as carbon nanotubes (CNTs) have been conducted. An X-ray generator using carbon nanotubes has an electron emission mechanism different from that of a conventional thermionic emission type X-ray generator using a tungsten filament. Since the electric field emission type X-ray generator is capable of emitting electron by application of a voltage, the electric field emission type X-ray generator are capable of performing faster electron emission and control compared to the thermionic emission type X-ray generator. Accordingly, emission of X-rays in a pulse form of on/off is relatively easy, thereby enabling various applications such as capturing of X-ray videos and providing a possibility of relatively low-dose X-ray imaging.

Currently, X-ray imaging in a medical field is determined according to imaging conditions including a tube voltage, a tube current, a Focus-to-Film Distance (FFD), a dose, an imaging time, and so on. However, such imaging conditions are presented on the basis of a conventional X-ray imaging apparatus to which a thermionic emission type X-ray generator is applied. Therefore, when such imaging conditions are followed, there is a problem in that advantages of an electric field emission type X-ray generator capable of achieving low dose and high image quality through pulse driving cannot be fully utilized.

### Disclosure

### Technical Problem

Accordingly, the present disclosure has been made keeping in mind the above problems occurring in the related art, and an objective of the present disclosure is to provide an X-ray imaging apparatus including an electric field emission type X-ray generator advantageous for pulse driving, the X-ray imaging apparatus being configured such that characteristic values of an X-ray generator and an X-ray detector are utilized so that an image of the same quality is provided at a relatively low dose or an image of relatively high quality is provided at an equal or lower dose, compared to a thermionic X-ray generator.

### Technical Solution

In order to solve the problems described above, in the present disclosure, there is provided an X-ray imaging apparatus including: an imaging unit including an X-ray generator configured to irradiate a subject with X-rays and an X-ray detector configured to receive the X-rays that have passed through the subject and to generate projection data on a frame-by-frame basis; an input unit into which imaging information including at least one information selected from a tube voltage, a tube current, and an irradiation time is input; a storage unit configured to store generator irradiation information that includes at least one information selected from a rising time and a falling time of the X-ray generator, detector detection information that includes at least one information selected from a frame rate and a readout time of the X-ray detector, and dose-pulse-image quality relationship information representing a relationship among an X-ray dose, the number of X-ray pulses, and image quality of an X-ray image; and a control unit configured to calculate, according to the imaging information, the number of X-ray pulses for X-ray imaging of the subject by using the generator irradiation information, the detector detection information, and the dose-pulse-image quality relationship information.

The control unit may be configured to calculate an X-ray pulse rate of the X-ray generator according to the number of X-ray pulses, may be configured to adjust the frame rate of the X-ray detector according to the X-ray pulse rate, and may be configured to control the X-ray generator and the X-ray detector such that the subject is X-ray imaged at the X-ray pulse rate and the adjusted frame rate during the irradiation time.

An X-ray dose according to the number of X-ray pulses may be equal to or less than the X-ray dose according to the imaging information.

The imaging information may further include mode information for selecting a low dose mode and a high image quality mode.

The number of pulses of the X-ray generator in the low dose mode may be smaller than the number of pulses of the X-ray generator in the high image quality mode.

The generator irradiation information may further include an interval time between X-ray pulses.

The interval time may be equal to or larger than the readout time of the X-ray detector.

The image quality of the X-ray image may include an SNR value.

### Advantageous Effects

According to the configuration of the present disclosure, in the X-ray imaging apparatus including the electric field emission type X-ray generator advantageous for pulse driving, characteristic values of the applied X-ray generator and the X-ray detector are utilized so that X-ray imaging based on high speed pulse driving of X-rays provides an image of the same quality at a relatively low dose or provides an image of relatively high quality at an equal or lower dose.

### Description of Drawings

FIG. 1 is a graph showing a theoretical relationship between the number of X-ray pulses and image quality under the same X-ray dose condition.
FIG. 2 is a view illustrating a result in which image quality is increased according to an increase in the number of X-ray pulses under the same X-ray dose condition in an actual apparatus.
FIG. 3 is a view illustrating a waveform of an X-ray dose according to pulse driving in an X-ray imaging apparatus according to an embodiment of the present disclosure.
FIG. 4 is a block diagram illustrating a configuration of the X-ray imaging apparatus according to an embodiment of the present disclosure.
FIG. 5 is a flowchart illustrating an X-ray imaging method of the X-ray imaging apparatus according to an embodiment of the present disclosure.

### Mode for Invention

Hereinafter, various embodiments of the present disclosure will be described with reference to the drawings. Through the embodiments, a technical concept of the present disclosure may be more clearly understood. The present disclosure is not limited to the embodiments described below.

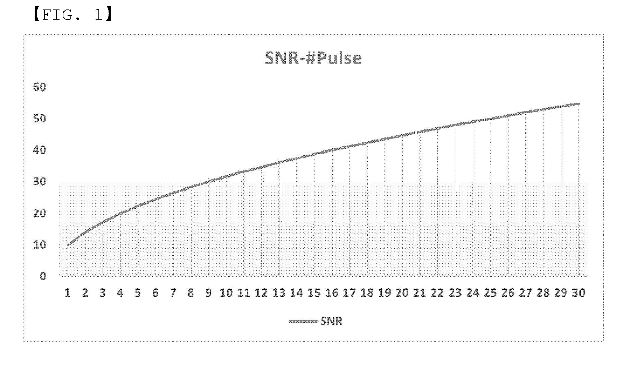

FIG. 1 is a graph showing a theoretical relationship between the number of X-ray pulses and image quality under the same X-ray dose condition.

An x-axis of the graph represents the number of X-ray pulses used to irradiate photons acquired by evenly dividing a total number of photons when the total number of photons is 100, and a y-axis of the graph represents a Signal-to-Noise Ratio (SNR) that is an index of image quality. X-rays statistically follow a Poisson distribution. When this characteristic is considered, an SNR value that is an index of image quality of an X-ray image is theoretically proportional to a square root of the number of photons.

That is, in a situation in which an X-ray dose is defined as the number of photons N (X-ray dose = the number of photons = N), when 100 photons are continuously irradiated, an SNR is root(100) = 10, and when 100 photons are divided into two pulses and 50 photons are irradiated in each pulse, root(50) = 7.07, and an SNR is 2 × 7.07 = 14.1, and thus, a theoretical result shows that an SNR value is increased by about 40% when the same photons are used in two pulses compared to when 100 photons are used in a single irradiation. According to this principle, under a condition that a total number of photons of X-rays is constant, it may be understood that image quality of an X-ray image may be increased according to a trend as shown in the graph as the number of pulses for irradiation increases.

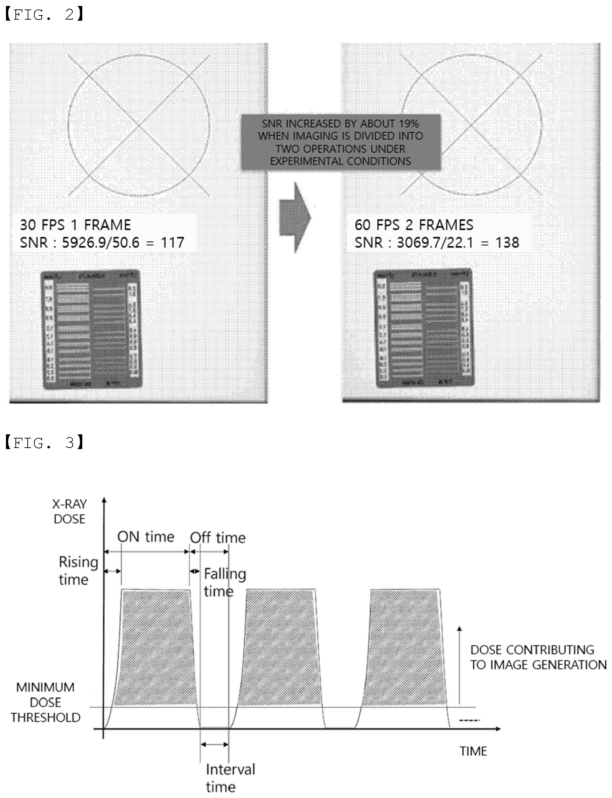

FIG. 2 is a view illustrating a result in which image quality is increased according to an increase in the number of X-ray pulses under the same X-ray dose condition in an actual apparatus.

In an actual X-ray imaging apparatus, although an increase in an SNR value, which is an image quality index, according to an increase in the number of X-ray pulses under the same X-ray dose condition does not exactly match the above-described theoretical increase trend due to various factors, when an SNR value of an image acquired by a single exposure at 30 Frames Per Second (FPS) and an SNR value of an image acquired by two exposures at 60 FPS under a condition satisfying the same dose are compared by using an actual product (EOX) of the applicant, it has been confirmed that the SNR is increased by about 19%.

FIG. 3 is a view illustrating a waveform of an X-ray dose according to pulse driving in an X-ray imaging apparatus according to an embodiment of the present disclosure.

When an electric field emission type X-ray generator is driven by using a square wave signal in the X-ray imaging apparatus according to the present embodiment, an X-ray irradiation dose rises to a target value with a predetermined rising time from a time point at which a driving signal is turned on, and falls with a predetermined falling time from a time point at which the driving signal is turned off, as illustrated in the graph. The rising time and the falling time may vary according to characteristics of the X-ray generator. In a case of an electric field emission type X-ray generator, although the rising time and the falling time are significantly shorter than those of a thermionic emission type X-ray generator, the rising time and the falling time may vary slightly according to individual characteristics among electric field emission type X-ray generators.

In the actual X-ray imaging apparatus, the presence of the rising time and the falling time is one of the causes of a difference between an increase in an SNR value according to an increase in the number of pulses under the same X-ray dose condition and a theoretically estimated value. Another cause is that, as illustrated in the drawing, only an X-ray irradiation dose higher than a minimum dose criterion, which is a value related to efficiency of the imaging apparatus, contributes to generation of an X-ray image. Due to such various factors, an increase in an SNR value according to the number of pulses under the same X-ray dose condition does not match a theoretical estimation. However, it has been confirmed through the actual product as described above that the SNR value increases according to an increase in the number of pulses.

In the drawing, an interval time represents an idle period during pulse driving of the X-ray generator. As illustrated in the drawing, the interval time may be inserted between X-ray pulses, or may be periodically inserted between a group of a plurality of pulses and a next group. The interval time may be equal to or longer than a readout time of the X-ray detector. In addition, the interval time may be inserted so that a pulse driving frequency of the X-ray generator matches a frame rate of the X-ray detector, i.e., the number of frames acquired per second.

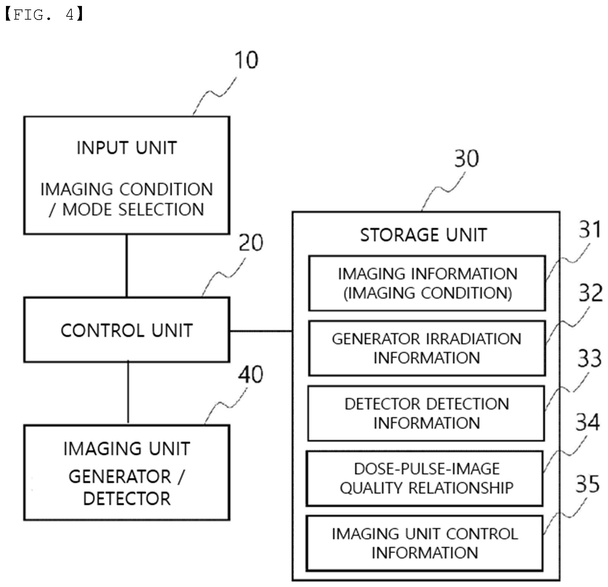

FIG. 4 is a block diagram illustrating a configuration of the X-ray imaging apparatus according to an embodiment of the present disclosure.

The X-ray imaging apparatus according to the present disclosure includes: an imaging unit 40 including an X-ray generator (hereinafter, referred to as a generator) and an X-ray detector (hereinafter, referred to as a detector); a control unit 20 configured to control X-ray imaging by controlling the generator and the detector; an input unit 30 configured to receive imaging conditions and an imaging mode selection from an operator; and a storage unit 30 configured to store various types of information 31, 32, 33, and 34 required for generating a control signal of the control unit 20. For example, the generator may be an electric field emission type X-ray generator including a carbon nanotube (CNT) emitter.

As one of the various types of information, imaging information 31 for each imaging type of a radiological examination is stored in the storage unit 30. The imaging information 31 may include imaging conditions such as a tube voltage, a tube current, an irradiation time, an X-ray dose (mAs: tube current × time), an imaging distance, and so on for each type of radiological examination that may be performed by using the X-ray imaging apparatus according to the present embodiment.

As one of the various types of information, generator irradiation information 32 is stored in the storage unit 30. The generator irradiation information 32 may include at least one information selected from a rising time, a falling time, and an interval time of the generator mounted in the X-ray imaging apparatus. For example, the generator irradiation information 32 may include the above-described rising time and the above-described falling time as characteristic values of the generator identified in a process of initially setting the X-ray imaging apparatus. When two or more generators are mounted in the X-ray imaging apparatus, the generator irradiation information 32 for each generator is stored in the storage unit 30.

As one of the various types of information, detector detection information 33 is stored in the storage unit 30. The detector detection information 33 may include at least one information selected from a frame rate and a readout time of the detector mounted in the X-ray imaging apparatus. The frame rate means a speed at which the detector acquires image data, i.e., the number of image frames per second, and the readout time means a time required to transmit image data of one frame to the control unit 20. When two or more detectors are mounted in the X-ray imaging apparatus, the detector detection information 33 for each detector is stored in the storage unit 30.

In addition, as one of the various types of information, dose-pulse-image quality relationship information 34 may be stored in the storage unit 30. For various X-ray dose values included in the imaging information 31, information on a relationship of an image quality index (for example, SNR) of an image acquired by the detector when irradiation is performed by dividing into a plurality of pulses having a predetermined tube voltage value and a predetermined tube current value may be included in the dose-pulse-image quality relationship information 34. The predetermined tube voltage value and the predetermined tube current value may be a plurality of values. The dose-pulse-image quality relationship information 34 may be provided and utilized as a kind of look-up table. The dose-pulse-image quality relationship information 34 may be prepared in a manner of representing an X-ray dose and image quality according to the number of pulses when irradiation of X-ray pulses are performed at a predetermined pulse rate (the number of pulses per second). The predetermined pulse rate may have a plurality of levels, and may be equal to or higher than the frame rate of the detector included in the detector detection information 33. In other words, one or a plurality of pulses may be configured to correspond to one frame of the detector.

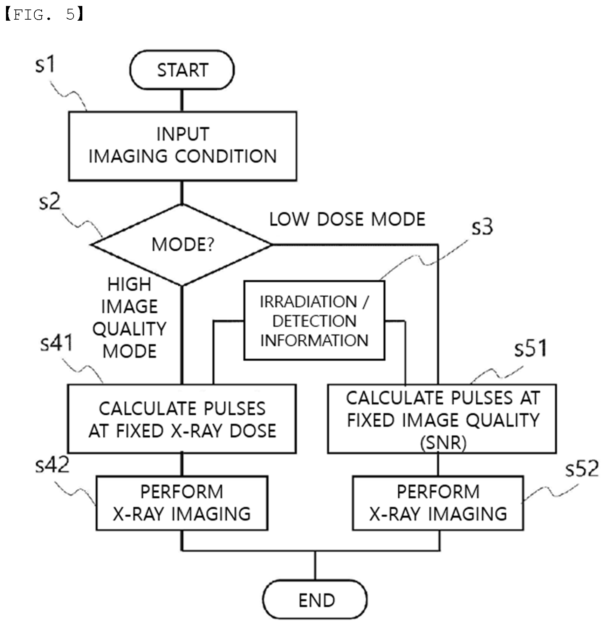

FIG. 5 is a flowchart illustrating an X-ray imaging method of the X-ray imaging apparatus according to an embodiment of the present disclosure.

Here, with reference to FIG. 4 and this drawing, a description is given of how the control unit 20 is configured to control the X-ray imaging apparatus according to the present embodiment by using the information stored in the storage unit 30.

When X-ray imaging starts, the control unit 20 receives imaging conditions through the input unit 10 according to an X-ray image to be captured (s1). For example, when an X-ray image corresponding to any one of standardized radiological examinations is captured, an operator may select a target body region, thereby substituting for input of the imaging conditions. Here, the control unit 20 may load imaging conditions corresponding to relevant imaging conditions from the imaging information 31 stored in the storage unit 30, and the operator may adjust at least some of the loaded imaging conditions through an additional input.

In addition, the imaging condition input process (s1) may be configured such that the operator selectively inputs any one mode selected from a plurality of imaging modes, including a low dose mode and a high image quality mode, through the input unit 10. According to a mode selected by the operator, the control unit 20 may calculate imaging unit control information 35 for controlling the imaging unit 40 during X-ray imaging by using the previously input imaging conditions, the generator irradiation information 32, the detector detection information 33, and the dose-pulse-image quality relationship information 34 that are stored in the storage unit 30 (s41 and s51). Here, the calculation includes not only direct computation but also retrieval of an appropriate value from a look-up table.

According to the imaging mode selection process (s2), when the operator selects the high image quality mode, the imaging unit control information 35 for driving the generator and the detector may be calculated such that, under a condition that the same X-ray dose as that of the imaging conditions input by the operator is applied, the corresponding dose is divided into a plurality of pulses, irradiation is performed, and projection data is acquired (s41). In this case, the control unit 20 may limit the number of X-ray irradiation pulses by setting an upper limit on an SNR value of an X-ray image in the high image quality mode such that an expected SNR value of the X-ray image within a range of the corresponding X-ray dose does not exceed the upper limit. The high image quality mode may be further subdivided into a general high image quality mode and an ultra-high image quality mode. In addition, projection data generated by a plurality of X-ray pulses while forming an X-ray image during imaging may be accumulated, a degree of increase in an SNR value of the X-ray image may be monitored, and X-ray pulse irradiation may be stopped when the SNR value reaches a target value of the high image quality mode.

According to the imaging mode selection process (s2), when the operator selects the low dose mode, the imaging unit control information for providing an X-ray image having the same image quality (SNR) as that of the imaging conditions input by the operator at a dose lower than an X-ray dose of the corresponding imaging conditions may be calculated (s51). The control unit 20 calculates the imaging unit control information 35 for controlling the imaging unit 40 during X-ray imaging by using the previously input imaging conditions, the generator irradiation information 32, the detector detection information 33, and the dose-pulse-image quality relationship information 34 that are stored in the storage unit 30.

Whereas the imaging conditions loaded from the imaging information 31 are based on a conventional X-ray generator that performs continuous X-ray irradiation, in the X-ray imaging apparatus according to the present disclosure, since an SNR of an image may be increased by performing X-ray irradiation in a pulse waveform as described above, even when a tube current or a total sum of X-ray pulse irradiation times (mAs) is reduced as compared with "tube current × time" (mAs) according to the imaging conditions to reduce an X-ray dose applied to a subject, a level of image quality required in general X-ray imaging may be satisfied. Accordingly, in the imaging unit control information 35 according to the low dose mode, at least one value selected from a tube current and a time has a value smaller than that included in the imaging information 31. When the generator and the detector are controlled at a maximum frame rate considering the generator irradiation information 32 and the detector detection information 33 and at a pulse rate suitable therefor, the imaging unit control information 35 in the low dose mode may be calculated in a manner of calculating the minimum number of pulses that satisfies an image quality (SNR) condition required for a corresponding radiological examination image.

When the imaging unit control information 35 is calculated as described above (s41 and s51), the control unit 20 stores the imaging unit control information 35 in the storage unit 30 at least temporarily, and controls the imaging unit 40 by using the imaging unit control information 35 so as to perform X-ray imaging according to each imaging mode (s42 and s52).

### Industrial Applicability

The present disclosure relates to an X-ray imaging apparatus, and may be used in a medical X-ray imaging apparatus and an X-ray imaging apparatus for non-destructive inspection for industrial use.

## Claims

1. An X-ray imaging apparatus comprising:
an imaging unit comprising an X-ray generator configured to irradiate a subject with X-rays and an X-ray detector configured to receive the X-rays that have passed through the subject and to generate projection data on a frame-by-frame basis;
an input unit into which imaging information comprising at least one information selected from a tube voltage, a tube current, and an irradiation time is input;
a storage unit configured to store generator irradiation information that comprises at least one information selected from a rising time and a falling time of the X-ray generator, detector detection information that comprises at least one information selected from a frame rate and a readout time of the X-ray detector, and dose-pulse-image quality relationship information representing a relationship among an X-ray dose, the number of X-ray pulses, and image quality of an X-ray image; and
a control unit configured to calculate, according to the imaging information, the number of X-ray pulses for X-ray imaging of the subject by using the generator irradiation information, the detector detection information, and the dose-pulse-image quality relationship information.

2. The X-ray imaging apparatus of claim 1, wherein the control unit is configured to calculate an X-ray pulse rate of the X-ray generator according to the number of X-ray pulses, is configured to adjust the frame rate of the X-ray detector according to the X-ray pulse rate, and is configured to control the X-ray generator and the X-ray detector such that the subject is X-ray imaged at the X-ray pulse rate and the adjusted frame rate during the irradiation time.

3. The X-ray imaging apparatus of claim 1, wherein an X-ray dose according to the number of X-ray pulses is equal to or less than the X-ray dose according to the imaging information.

4. The X-ray imaging apparatus of claim 1, wherein the imaging information further comprises mode information for selecting a low dose mode and a high image quality mode.

5. The X-ray imaging apparatus of claim 4, wherein the number of pulses of the X-ray generator in the low dose mode is smaller than the number of pulses of the X-ray generator in the high image quality mode.

6. The X-ray imaging apparatus of claim 1, wherein the generator irradiation information further comprises an interval time between X-ray pulses.

7. The X-ray imaging apparatus of claim 6, wherein the interval time is equal to or larger than the readout time of the X-ray detector.

8. The X-ray imaging apparatus of claim 1, wherein the image quality of the X-ray image comprises an SNR value.
